# EUROPEAN PATENT APPLICATION

(11) **EP 0 903 144 A1**
(43) Date of publication of application: **24.03.1999**
(21) Application number: 97908517.2
(22) Date of filing: 27.03.1997
(51) Int. Cl.: A61K 31/40, A61K 31/495, C07D 209/90, C07D 471/06, C07D 487/06

(54) **REMEDY FOR RETINAL NEUROPATHY**

(30) Priority: 17.04.1996 JP 120957/96
(71) Applicant: SUMITOMO PHARMACEUTICALS COMPANY, LIMITED, Osaka-shi, Osaka 541 (JP)
(72) Inventor: IKEDA, Kazuhito, 15-10, Kusunoki-cho Ashiya-shi Hyogo 659 (JP); TATSUNO, Tohru, Kobe-shi Hyogo 651-24 (JP); TANAKA, Hiroyasu, Hyogo 661 (JP)
(74) Representative: Cresswell, Thomas Anthony
(86) International application number: JP9701037
(87) International publication number: WO9738691

(57) **Abstract**

The present invention provides a medicament for treating retinal neuropathy, containing a tricyclic nitrogen-containing compound which is an N-methyl-D-aspartate receptor antagonist or a pharmaceutically acceptable salt thereof.

## Description

### Technical Field

The present invention relates to a medicament for treating retinal neuropathy, containing a tricyclic nitrogen-containing compound which is an N-methyl-D-aspartate receptor antagonist or a pharmaceutically acceptable salt thereof.

### Background Art

It has been reported that ischemia participates in several neurodegenerative disorders in the central and peripheral nervous systems. It is also supposed that ischemic damage may cause retinal neuropathy including glaucoma, central retinal artery occlusion, branch retinal artery occlusion, central retinal vein occlusion, branch retinal vein occlusion, ischemic optic neuropathy, diabetic retinopathy, macular degeneration, retinopathy of prematurity, etc. ("Ophthalmology New Insight" Vol.6 Eye Circulation (1995) Medical View Co., Ltd.).

### (1) Glaucoma

Glaucoma affects 3.5% of persons who are older than 40 years. It is assumed that there are 1,920,000 patients in Japan, and 105,000,000 patients in the world (Drug & Market Development, Vol.5, P.272 (1995)). Glaucoma is basically characterized by an elevated intraocular pressure (IOP), at rophy of the retina and excavation of the optic nerve head. Many do not recuperate satisfactorily, and would become blind in the worst case. It is reported that glaucoma ranks 4th as the cause of blindness in Japan and 120,000 patients become blind in a year in the United States of America.

The mechanisms of optic nerve damage are assumed to involve mechanical theory and vascular theory ("Ophthalmology Diagnosis Practice" Vol.10 Method for diagnosing glaucoma p.78 (1994) Bun-eido Co.,Ltd.). The former suggests that the depression of the optic nerve head by elevated IOP results in axonal damage in the optic nerve with ensuing blockade of axonal transport followed by axonal death. The latter proposes that the pressure to the optic nerve head interferes with the blood flow in the central retinal artery and vein which are going through the optic nerve head and as a result the nerve cells which are supplied with oxygen and nutrients from the vessel are damaged. The vascular theory is supported by the data that a decrease in the blood vessel network and a leakage of fluorescent dye are observed in glaucoma by fluorescein angiography, and that glaucoma-like optic nerve head depression and visual field deficiency are observed in anterior ischemic optic neuropathy, in which the central retinal artery is clearly damaged.

Many drugs for reducing IOP have been developed, because glaucoma is basically characterized by elevated IOP. According to their mechanisms of action, they are classified into 5 groups: parasympathomimetic drugs, sympathomimetic drugs, β-blockers, carbonic anhydrase inhibitors and hyperosmotic agents, all of which show a medical effect by reducing IOP ("Chemotherapy of Glaucoma" (1990) Mikusu Co., Ltd.). However, patients who do not recuperate well and suffer from optic nerve damage still remain, even if the drug effectively reduces IOP. In addition, it has been reported that there are many glaucoma patients without elevated IOP, that is, with low IOP glaucoma. It is assumed that there are 1,000,000 patients with low IOP glaucoma in the United States of America, as compared with 2,000,000-3,000,000 patients with elevated IOP (Drug & Market Development, Vol.5, p.272 (1995)). Therefore, a drug not for reducing IOP but for directly protecting the optic nerve cells is desired. According to the vascular theory as described above, a drug for alleviating the toxicity of excitatory amino acids in the ischemic condition would be applicable.

### (2) Central retinal artery occlusion

Central retinal artery occlusion occurs by a thrombus forming at the passing point of the central retinal artery through the lamina cribrosa. Visual acuity in one eye declines drastically, and optic nerve atrophy is observed. Unlike chronic ischemia, neovascularization is not recognized afterwards. When an acute ischemic state continues for 30-40 minutes, the retina degenerates irreversibly and this results in necrosis because the central retinal artery is the end artery. Therefore, in the case of complete occlusion, recovery of visual acuity would not be expected ("Illustration Ophthalmology" 4th edition, p.232 (1992) Buneido Co., Ltd.).

As to treatment, immediately after its detection eyeball massage is performed to resume blood flow. As to medication, urokinase is administered together with dextran to decompose the thrombus, and prostaglandin E₁ (Alprostadil) is administered to prevent thrombus formation ("Ophthalmology Medication Handbook" p. 75 (1992) Mikusu Co., Ltd.).

### (3) Branch retinal artery occlusion

Branch retinal artery occlusion occurs by a thrombus forming at the branch artery in the eye, and injury is dominated only in the region. Treatment is carried out in a manner similar to that of central retinal artery occlusion.

### (4) Central retinal vein occlusion

Central retinal vein occlusion occurs by a thrombus forming at the lamina cribrosa, and is classified into hemorrhagic retinopathy and venous stasis retinopathy according to the existence or absence of hemorrhage ("Illustration Ophthalmology" 4th edition, p.236 (1992) Bun-eido Co., Ltd.).

Hemorrhagic retinopathy mostly affects old people, and arteriosclerosis is the cause in more than half of the cases. Flame-shaped hemorrhage occurs on the retinal epidermis around the optic disk along the nerve fiber followed by a drastic decline of visual acuity.

As to treatment, urokinase is administered together with dextran in a similar manner to the case of central retinal artery occlusion, and carbazochrome sodium sulfonate or adrenochromeguanylhydorazone mesylate is administered to strengthen the vascular vessels. Laser photocoagulation is performed to prevent macular edema or neovascularization. ("Ophthalmology Medication Handbook" p.75 (1992) Mikusu Co., Ltd.).

As to venous stasis retinopathy, two types are known. One is caused by inflammation and the other is caused by arteriosclerosis. The former mostly affects younger people and the latter affects older people. Venous stasis retinopathy is characterized by severe expansion and meandering of the vein and rubedo of the optic nerve head. However, the decline in visual acuity is less severe compared to that in hemorrhagic retinopathy.

It is treated with a medicine similar to that for hemorrhagic retinopathy. Laser photocoagulation is not performed ("Illustration Ophthalmology" 4th edition, p.238 (1992) Bun-eido Co., Ltd.).

### (5) Branch retinal vein occlusion

The cause and the symptoms are similar to that of central retinal vein occlusion, but the frequency of occurrence is 3 times more. Occlusion mostly occurs at the superior auricular chiasma of artery and vein, and it mostly affects old people ("Illustration Ophthalmology" 4th edition, p.238 (1992) Bun-eido Co., Ltd.).

### (6) Ischemic optic neuropathy

Ischemic optic neuropathy is a disease with optical disfunction caused by nutritive vessel occlusion and subsequent ischemic necrosis of the optic nerve. It is called anterior ischemic optic neuropathy, because the blockade of blood flow is liable to occur at the lamina cribrosa, which results in damage of the optic nerve head ("Illustration Ophthalmology" 4th edition, p.334 (1992) Bun-eido Co., Ltd.).

Clinically, a sudden decline in visual acuity in one eye is observed without premonitory symptoms in middle-aged or older people. The optic nerve head becomes pale and edematous, frequently accompanied by flame-shaped hemorrhage. Horizontal hemianopia is characteristically observed, and bow-shaped scotoma and central scotoma are also observed.

The causes are temporal arteritis or idiopathic ischemic optic neuropathy. The former is scarce, and the latter occurs in most cases. Idiopathic ischemic optic neuropathy is derived from systemic disorders such as arteriosclerosis, hypertention, diabetes, etc.

As to treatment, only medication is applied. Steroids such as prednisolone are administered to alleviate edema. Ocular hypotensive agents such as carbonic anhydrase inhibitors(e.g. acetazolamide) are administered to improve blood flow around the lamina cribrosa. Vitamin B₁ or B₁₂ is administered for nerve invigoration ("Ophthalmology Medication Handbook" p.85 (1992) Mikusu Co., Ltd.).

### (7) Diabetic retinopathy Diabetic retinopathy occurs by the occlusion of retinal

microvessels resulting from degeneration and necrosis of the endothelial cells in capillary vessels, thrombosis formation, and acceleration of blood coagulation after continuous hyperglycemia over several years. This disease is classified into 3 stages: simple diabetic retinopathy, preproliferative diabetic retinopathy and proliferative diabetic retinopathy, according to its pathological developments ("Clinical Diabetic Retinopathy" (1994) Saishin-igaku Co., Ltd.).

Treatment of simple diabetic retinopathy is at first systemic blood sugar control, and in cases where hemorrhage is detected, a vessel reinforcing agent and streptokinase and streptodornase (varidase) are applied. In preproliferative diabetic retinopathy, panretinal photocoagulation is applied. In proliferative diabetic retinopathy, vitrectomy is applied in cases where tractile retinal detachment occurs ("Ophthalmology Medication Handbook" p.76 (1992) Mikusu Co., Ltd.). There is no drug for protecting the retinal nerve, and only tocopherol calcium succinate (vitamin E) and tocopherol acetate (Juvela) are used as supporting drugs.

Diabetic retinopathy occurs about 10 years after occurrence of diabetes, and affects 39 % of patients of insulin-dependent type and 47 % of those of insulin-independent type. It is assumed that there are about 600,000 patients in Japan. It ranks first as the cause of blindness. A therapeutic drug is highly needed socially, and the development of a new drug is desired.

The relation between ischemia and diabetic retinopathy is explained as follows. A disorder in glucose metabolism leads to the disappearance of retinal capillary mural cells, hyperplasia of basement membrane, the degeneration of endothelial cells, deposits of glycogen, mucopolysaccharide and glycoprotein, microaneurysm, hemorrhage and exudative changes. These pathological changes results in the non-perfusion of capillary vessels, which produces hypoxic and ischemic conditions. Therefore, the development of a new drug for alleviating ischemic damage is expected.

### (8) Macular degeneration

The macula, a part of the retina, is located in the center of the visual field, which is critical for visual acuity. Macular degeneration is the general term for disorders in this area. There are five main disorders: central serous chorioretinopathy, central exudative chorioretinopathy, senile disciform macular degeneration, senile atrophic macular degeneration and idiopathic vitreoretinal interface maculopathy ("Illustration Ophthalmology" 4th edition, p. 252-255 (1992) Bun-eido Co., Ltd.).

Central serous chorioretinopathy and central exudative chorioretinopathy are diseases in which serum or blood from choroid flows through the injured pigment epithelium and is reserved in the subretinal space.

Senile disciform macular degeneration is a disease in which exudative change, hemorrhage and choroidal neovascularization occur in the macula. Senile atrophic macular degeneration is characterized by no exudative changes and atrophy of retinal pigment epithelial cells.

Idiopathic vitreoretinal interface maculopathy is a disease in which a transparent or semi transparent epiretinal membrane is formed in the macula and vessels meander in the macula.

It is supposed that ischemic retinal damage participates in all diseases except for senile atrophic macular degeneration because these diseases are caused by vascular damage.

As to treatment, photocoagulation is fundamentally applied in the case of neovascularization. As to medication, hyperosmotic agents such as isosorbide and glycerin are applied for the absorption of subretinal fluid. Kallidinogenase is applied for the improvement of choroidal circulation. Streptokinase and streptodornase (varidase) are applied for vascular dilation. Vitamins, steroids, etc. are also applied ("Ophthalmology Medication Handbook" p. 77-78 (1992) Mikusu Co., Ltd.). However, there is no drug for acting on retinal nerve cells, and the development of a new drug is desired.

### (9) Retinopathy of prematurity

Retinopathy of prematurity arises from hypoxia after the provision of preterm infants with high concentration of oxygen, which induces occlusive change in the peripheria of immature retinal vascular vessels.

It is classified into 2 types: Type I in which spontaneous recovery is expected and Type II which mainly affects very small immature infants with little tendency for spontaneous recovery or satisfactory recuperation ("Illustration Ophthalmology" 4th edition, p. 248-251 (1992) Bun-eido Co., Ltd.).

As to treatment, in Type I, cryocoagulation is applied to the patients who have distinct exudation to vitreous. In Type II, photocoagulation or cryocoagulation is applied immediately after diagnosis.

There is no drug for the treatment, and therefore the development of a drug for alleviating ischemic damage is desired.

JP 7-188166, A (EP-657427), JP 7-2855, A (EP-627434) and JP 7-500591,A (WO 93/08188) report that tricyclic nitrogen-containing compounds are N-methy-D-aspartate receptor antagonists, but does not disclose that these compounds are effective against retinal neuropathy.

### Description of the invention

The present invention is intended to provide a medicament for treating retinal neuropathy.

The present inventors found that a tricyclic nitrogen-containing compound which is an N-methyl-D-aspartate receptor antagonist, or a pharmaceutically acceptable salt thereof, prevents the death of retinal neuronal cells associated with ischemia, and is useful as a medicament for treating retinal neuropathy. Thus, the present invention has been accomplished.

That is, the present invention is as follows.
[1] A medicament for treating retinal neuropathy, containing a tricyclic nitrogen-containing compound which is an N-methyl-D-aspartate receptor antagonist or a pharmaceutically acceptable salt thereof.
[2] The medicament of [1], wherein the tricyclic nitrogen-containing compound which is an N-methyl-D-aspartate receptor antagonist, is a compound represented by figure 1, 2 or 3: 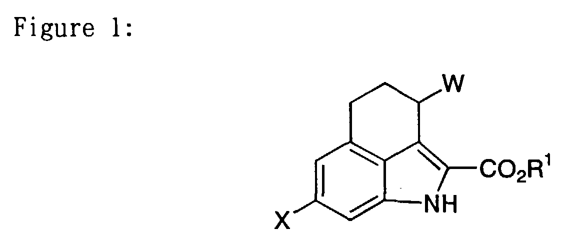 wherein
   X is alkyl, halogen or cyano;
   R¹ is hydrogen or a carboxyl protecting group;
   W is hydrogen, -CO₂R³ⁱ, -CONR³ⁱR⁴ⁱ, -A-CO₂R³ⁱ or -A-CONR³ⁱR⁴ⁱ, wherein-A-is alkylene, and R³ⁱ and R⁴ⁱ are independently hydrogen, alkyl, aryl or substituted aryl. 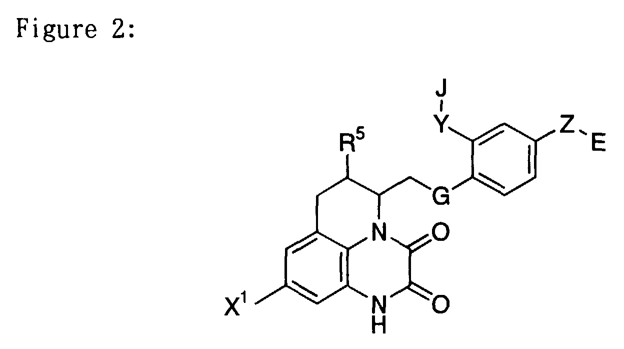
   wherein
   X¹ is hydrogen, alkyl, halogen, cyano, trifluoromethyl or nitro;
   R⁵ is hydrogen, alkyl, cycloalkyl or cycloalkylalkyl;
   G is -CONR⁶- or -NR⁶CO-, wherein R⁶ is hydrogen or alkyl;
   J is an acidic group or a group which is convertible thereto *in vivo*;
   Y is a single bond, alkylene, alkenylene, substituted alkylene or -Y¹-Q-Y²-, wherein Y¹ is a single bond or alkylene, Y² is alkylene, Q is oxygen or sulfur;
   E is a basic group or a group which is convertible thereto *in vivo*;
   Z is alkylene. 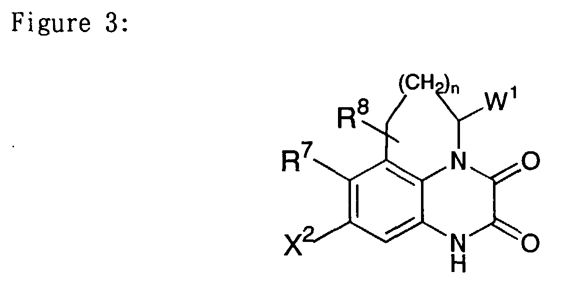
   wherein
   X² is alkyl, halogen, cyano, trifluoromethyl, nitro, hydroxy, amino, alkylamino, alkoxy, alkanoyl, alkoxycarbonyl, sulfamoyl, carbamoyl, alkylcarbamoyl, alkylthio, alkylsulfinyl, alkylsulfonyl, alkylsulfamoyl, alkylsulfonylamino or acylamino;
   R⁷ is hydrogen, alkyl, halogen, cyano, trifluoromethyl, nitro, hydroxy, amino, alkylamino, alkoxy, alkanoyl, alkoxycarbonyl, sulfamoyl, carbamoyl, alkylcarbamoyl, alkylthio, alkylsulfinyl, alkylsulfonyl, alkylsulfamoyl, alkylsulfonylamino or acylamino;
   R⁸ is hydrogen, alkyl, cycloalkyl, alkenyl, alkynyl, cycloalkylalkyl, arylalkyl, substituted arylalkyl, aryl or substituted aryl;
   W¹ is hydrogen, -CO₂R⁹, -CO₂Y³, -CONR⁹R¹⁰, -CONR⁹Y³, -CON(OR⁹)R¹⁰, - COR⁹, cyano, tetrazolyl or substituted alkyl;
   R⁹ and R¹⁰ are independently hydrogen, alkyl, cycloalkyl, alkenyl, alkynyl, cycloalkylalkyl, arylalkyl, substituted arylalkyl, aryl, substituted aryl, heteroaryl, substituted heteroaryl, heteroarylalkyl, substituted heteroarylalkyl or heterocycloalkyl;
   Y³ is mono-substituted alkyl or di-substituted alkyl; n is 0 or 1.
[3] The medicament of [2], wherein the tricyclic nitrogen-containing compound which is an N-methyl-D-aspartate receptor antagonist, is a compound represented by figure 1.
[4] The medicament of [1], [2] or [3], for treating retinal neuropathy associated with ischemia.
[5] The medicament of [1], [2] or [3], for treating glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.
[6] A method of treating retinal neuropathy which comprises administering a tricyclic nitrogen-containing compound which is an N-methyl-D-aspartate receptor antagonist or a pharmaceutically acceptable salt thereof.
[7] A pharmaceutical composition for treating retinal neuropathy, containing a tricyclic nitrogen-containing compound which is an N-methyl-D-aspartate receptor antagonist or a pharmaceutically acceptable salt thereof, and a pharmaceutically acceptable carrier.
[8] Use of a tricyclic nitrogen-containing compound which is an N-methyl-D-aspartate receptor antagonist or a pharmaceutically acceptable salt thereof, in the preparation of a medicament for treating retinal neuropathy.

A tricyclic nitrogen-containing compound which is an N-methyl-D-aspartate receptor antagonist, includes for example a compound represented by figure 1, 2 or 3, or a pharmaceutically acceptable salt thereof.

### A tricyclic nitrogen-containing compound represented by figure 1

A compound represented by figure 1 is described in JP 7-188166, A (EP-657427), together with its manufacturing methods.

The compound represented by figure 1 is described more specifically as following. 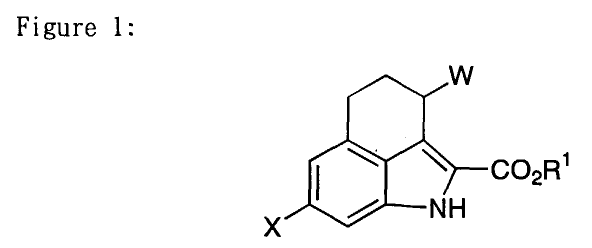 wherein X, R¹ and W are as defined above.

A preferred example of -A-CONR³ⁱR⁴ⁱ may be a group represented by figure 4. 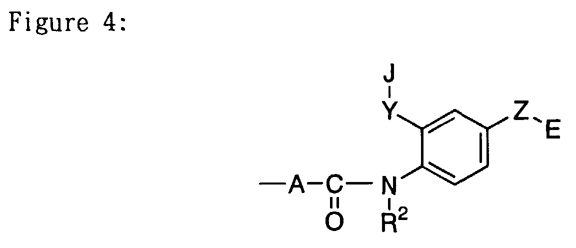 wherein A, R², J, E, Y and Z are as defined above.

In this compound, the term "alkyl" includes straight or branched C₁-C₆ alkyl. Typical examples are methyl, ethyl, n-propyl, isopropyl, sec-butyl, tert-butyl, neopentyl, n-pentyl and n-hexyl.

The term "halogen" includes fluorine, chlorine, bromine and iodine. A typical example is chlorine.

The term "aryl" includes C₆-C₁₀ aryl. Typical examples are phenyl, 1-naphthyl and 2-naphthyl.

The term "carboxyl protecting group" includes a group which is readily hydrolyzed *in vivo* to give hydrogen, and a protecting group which is used to prevent undesired side reactions during the synthesis of the desired compounds. The group which is readily hydrolyzed *in vivo* to give hydrogen includes alkyl and substituted alkyl. Alkyl is the same as mentioned above, and the substituent of substitued alkyl includes straight or branched C₁-C₆ alkoxy such as methoxy, ethoxy and tert-butoxy, straight or branched C₁-C₆ alkanoyloxy such as acetoxy, ethylcarbonyloxy and pivaloyloxy, and aroyloxy containing up to 11 carbon atoms such as benzoyloxy.

The protecting group which is used to prevent undesired side reactions during the synthesis includes substituted or unsubstituted benzyl such as benzyl, p-methoxybenzyl and p-nitrobenzyl in addition to alkyl and substituted alkyl as mentioned above.

The term "alkylene" as used in A includes straight or branched C₁-C₂ alkylene. Typical examples are methylene and methylmethylene. The most preferred example is methylene.

The substituent of substituted aryl includes alkyl, halogen, -Y-J and -Z-E, wherein Y, J, Z and E are as defined above. The number of the substitent(s) may be one or more which may be the same or different.

The term "basic group" includes a group which is readily protonated *in vivo* to provide a cation. Typical examples are -NH₂, -NHR^{3E}, -NR^{3E}R^{4E}, -NH-C(=NH)-NH₂, -NH-C(=NH)-NHR^{3E} and -NH-C(=NH)-NHR^{3E}R^{4E}, wherein R^{3E} and R^{4E} are independently alkyl, cycloalkyl, alkenyl or cycloalkylalkyl, or R^{3E} and R^{4E} are joined to form a cyclic amine together with the nitrogen atom.

The term "alkenyl" includes straight or branched C₃-C₆ alkenyl of which an olefinic carbon atom may not be connected directly with the nitrogen atom of the basic group. Typical examples are allyl, 2-butenyl and 3-butenyl.

The term "group which is convertible to a basic group *in vivo*" includes -NHL, -NLR^{3E}, -NH-C(=NL) -NH₂, -NH-C(=NL) -NHR^{3E} and -NH-C(=NL)-NHR^{3E}R^{4E} wherein R^{3E} and R^{4E} are as defined above, L is a hydrolyzable group *in vivo*, such as alkanoyl and alkoxycarbonyl.

The term "alkanoyl" includes straight or branched C₁-C₆ alkanyl. Typical examples are formyl, acetyl, propanoyl, n-butanoyl and pivaloyl.

The term "alkoxycarbonyl" includes straight or branched C₂-C₆ alkoxycarbonyl. Typical examples are methoxycarbonyl, ethoxycarbonyl, propoxycarbonyl, isopropoxycarbonyl, sec-butoxycarbonyl and tert-butoxycarbonyl.

The term "acidic group" includes a group which is readily deprotonated *in vivo* to provide an anion. Typical examples are carboxyl and tetrazolyl.

The term "group which is convertible to an acidic group *in vivo*" includes a group which generates an acidic group *in vivo* by hydrolysis. Typical examples are -COOR^{3J}, -CONH₂, -CON(OH)H, -CONHR^{3J}, -CON(OH)R^{3J}, -CON(OR^{3J})R^{4J} and -CONR^{3J}R^{4J}, wherein R^{3J} and R^{4J} are independently alkyl, cycloalkyl, alkenyl or cycloalkylalkyl, or R^{3J} and R^{4J} are joined to form a cyclic amine together with the nitrogen atom.

The cyclic amine which R^{3E} and R^{4E}, or R^{3J} and R^{4J} are joined to form includes 3- to 7-membered cyclic amines such as azetidine, pyrrolidine and piperidine, and 5- to 7-membered cyclic amines containing other heteroatoms such as oxygen and nitrogen as a ring member, such as piperidine, N-methylpiperidine and morpholine.

The term"alkylene" as used in figure 4 includes straight or branched C₁-C₆ alkylene. Typical examples are methylene, dimethylene, trimethylene, tetramethylene, 2-methyltrimethylene, 3-methyltrimethylene, 1,1-dimethylmethylene, pentamethylene and hexamethylene.

The term "alkenylene" includes straight or branched C₂-C₆ alkenylene. Typical examples are vinylene, 1-propenylene, 2-propenylene, 3-butenylene, 2-ethyl-3-butenylene, 4-pentenylene, 3-methy-4-pentenylene and 1-hexenylene.

The substituent of substituted alkylene for Y includes hydroxy, -OR^{3S}, -OCOR^{3S}, amino, -NHCOR^{3S}, -NHCO₂R^{3S}, carboxyl and -CO₂R^{3S}, wherein R^{3S} is alkyl, cycloalkyl, alkenyl or cycloalkylalkyl. Typical examples of substituted alkenylene are -CH(OH)-, -CH(OAc)-, -CH(CO₂-tert-Bu)- and -CH₂CH₂CH(CO₂Et)-. Preferably, the substituent and J group may be attached to the same carbon atom.

Typical examples of -Y¹-Q-Y²- are -OCH₂-, -SCH₂-, -CH₂OCH₂-, - CH₂SCH₂- and -CH₂CH₂OCH(CH₃)-.

### A tricyclic nitrogen-containing compound represented by figure 2

A compound represented by figure 2 is described in JP 7-2855, A (EP-627434), together with its manufacturing methods.

The compound represented by figure 2 is described more specifically as follows. 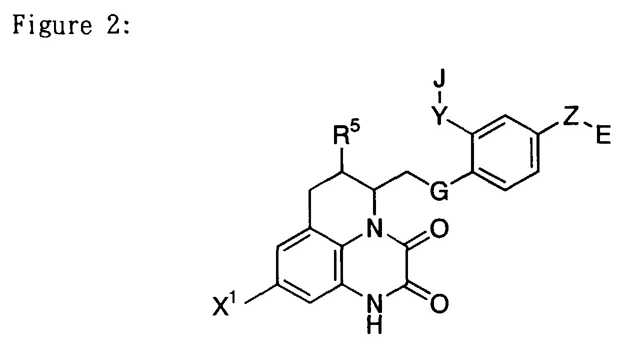 wherein X¹, R⁵, G, J, E, Y and Z are as defined above.

In this compound, the term "cycloalkyl" includes C₃-C₇ cycloalkyl. Typical examples are cyclopropyl, cyclobutyl, cyclopentyl, cyclohexyl, cycloheptyl and the like.

The term "cycloalkylalkyl" includes cycloalkylalkyl containing up to 13 carbon atoms. Typical examples are cyclopropylmethyl, cyclopentylethyl, cyclohexylmethyl, cyclohexylpropyl and the like.

The meanings of the other terms as used in this compound are the same as in the tricyclic nitrogen-containing compound represented by figure 1.

### A tricyclic nitrogen-containing compound represented by figure 3

A compound represented by figure 3 is described in JP 7-500591, A (WO 93/08188), together with its manufacturing methods.

The compound represented by figure 3 is described more specifically as follows. 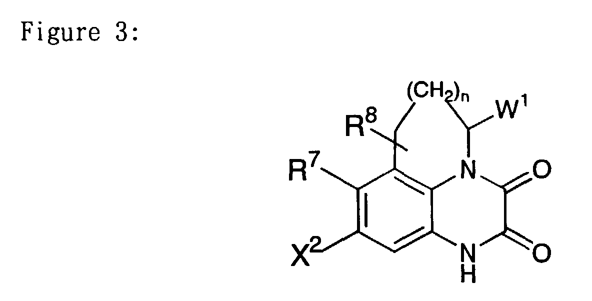 wherein X², R⁷, R⁸, W¹ and n are as defined above.

In this compound, the term "alkyl" includes straight or branched C₁-C₆ alkyl. Typical examples are methyl, ethyl, propyl, 2-propyl, butyl, 2-butyl, 3-methylpropyl, 1,1-dimethylethyl, pentyl, hexyl and the like.

The term "halogen" includes fluorine, chlorine, bromine, iodine and the like. Typical examples are chlorine and bromine.

The term "alkoxy" includes straight or branched C₁-C₆ alkoxy. Typical examples are methoxy, ethoxy, propoxy, 2-propoxy, butoxy, 1,1-dimethylethoxy, pentoxy, hexoxy and the like.

The term "alkanoyl" includes straight or branched C₁-C₆ alkanoyl. Typical examples are formyl, acetyl, propanoyl, 2-propanoyl, pivaloyl and the like.

The term "alkoxycarbonyl" includes straight or branched C₁-C₆ alkoxycarbonyl. Typical examples are methoxycarbonyl, ethoxycarbonyl, propoxycarbonyl, 2-propoxycarbonyl and the like.

The term "alkylthio" includes straight or branched C₁-C₆ alkylthio. Typical examples are methylthio, ethylthio, n-propylthio, 2-propylthio, sec-butylthio, tert-butylthio, neopentylthio, n-pentylthio, n-hexylthio and the like.

The term "alkylsulfinyl" includes straight or branched C₁-C₆ alkylsulfinyl. Typical examples are methylsulfinyl, ethylsulfinyl, propylsulfinyl, 2-propylsulfinyl and the like.

The term "alkylsulfonyl" includes straight or branched C₁-C₆ alkylsulfonyl. Typical examples are methylsulfonyl, ethylsulfonyl, propylsulfonyl, 2-propylsulfonyl and the like.

The term "alkylcarbamoyl" includes mono- or di-alkylcarbamoyl, wherein the alkyl moiety is straight or branched C₁-C₆ alkyl. Typical examples are methylcarbamoyl, methylethylcarbamoyl, diethylcarbamoyl, propylcarbamoyl, diisopropylcarbamoyl, hexylcarbamoyl and the like.

The term "alkylsulfamoyl" includes sulfamoyl substitued with 1 or 2 straight or branched C₁-C₆ alkyl(s). Typical examples are methylsulfamoyl, methylethylsulfamoyl, diethylsulfamoyl, propylsulfamoyl, diisopropylsulfamoyl, hexylsulfamoyl and the like.

The term "alkylsulfonylamino" includes straight or branched C₁-C₆ alkylsulfonylamino. Typical examples are methylsulfonylamino, ethylsulfonylamino, dimethylsulfonylamino, n-propylsulfonylamino, 2-propylsulfonylamino, sec-butylsulfonylamino, tert-butylsulfonylamino, neopentylsulfonylamino, n-pentylsulfonylamino and the like.

The term "acylamino" includes straight or branched C₁-C₆ alkanoylamino and C₇-C₁₁ aroylamino. Typical examples are formylamino, acetylamino, propanoylamino, butanoylamino, sec-butanoylamino, n-pentanoylamino, n-hexanoylamino, benzoylamino and the like.

The term "cycloalkyl" includes C₃-C₇ cycloalkyl. Typical examples are cyclopropyl, cyclobutyl, cyclopentyl, cyclohexyl, cycloheptyl and the like.

The term "alkenyl" includes straight or branched C₂-C₆ alkenyl. Typical examples are vinyl, allyl, 1-propenyl, 1-, 2- or 3-butenyl and the like.

The term "alkynyl" includes straight or branched C₂-C₆ alkynyl. Typical examples are ethynyl, propargyl, 1-propynyl, butynyl, pentynyl and the like.

The term "cycloalkylalkyl" includes straight or branched alkyl attached to cycloalkyl, which contains up to 13 carbon atoms. Typical examples are cyclopropylmethyl, cyclopentylethyl, cyclohexylmethyl, cyclohexylpropyl and the like.

The term "arylalkyl" includes straight or branched alkyl attached to aryl, which contains up to 15 carbon atoms. Typical examples are benzyl, phenylethyl, 1- or 2-naphthylmethyl, 1- or 2-naphthylpropyl and the like.

The term "aryl" includes aryl containing up to 10 carbon atoms. Typical examples are phenyl, 1-naphthyl, 2-naphthyl and the like.

The term "heteroaryl" includes 5- or 6-membered heteroaryl containing up to 4 nitrogen atoms, 5- or 6-membered heteroaryl containing up to 2 nitrogen atoms, up to 1 oxygen atom and/or up to 1 sulfur atom, and 5- or 6-membered heteroaryl containing up to 3 nitrogen atoms, up to 1 oxygen atom and/or up to 1 sulfur atom, which is fused with a benzene ring. Typical examples are pyridyl, thienyl, oxadiazolyl, imidazolyl, thiazolyl, isothiazolyl, oxazolyl, isoxazolyl, tetrazolyl, furyl, pyrrolyl, benzotriazolyl and the like.

The term "heteroarylalkyl" includes straight or branched C₁-C₆ alkyl attached to heteroaryl. Typical examples are pyridylmethyl, thienylmethyl, oxadiazolylmethyl, pyridylethyl, thienylethyl, oxadiazolylethyl, pyridylpropyl, thienylpropyl, oxadiazolylpropyl, imidazolylmethyl, thiazolylmethyl, isothiazolylmethyl, oxazolylmethyl, isoxazolylmethyl, tetrazolylmethyl, furylmethyl, pyrrolylmethyl and the like.

The term "heterocycloalkyl" includes heterocycloalkyl containing up to 6 carbon atoms together with 1 or 2 heteroatoms which are selected from nitrogen, oxygen and sulfur atoms, and heterocycloalkyl containing up to 10 carbon atoms together with 1 or 2 heteroatoms which are selected from nitrogen, oxygen and sulfur atoms, which is fused with a benzene ring. Typical examples are piperidyl, piperazinyl, morpholinyl, tetrahydropyranyl, tetrahydrofuranyl, pyrrolidinyl, dithianyl, indolyl, isoindolyl, tetrahydro-1-quinolinyl, and the like.

The term "alkylamino" includes mono- and di-alkylamino, wherein the alkyl group is straight or branched C₁-C₆ alkyl. Typical examples are methylamino, ethylamino, propylamino, 2-propylamino, butylamino, pentylamino, hexylamino, dimethylamino, diethylamino, dipropylamino, di-2-propylamino, dibutylamino, dipentylamino, dihexylamino and the like.

The alkyl of the term "substituted alkyl" as used in W¹ includes straight or branched C₁-C₄ alkyl. Typical examples are methyl, ethyl, propyl, 2-propyl, butyl and the like.

The substituent of the term "substituted alkyl" as used in W¹ includes CO₂R⁹, CO₂Y³, CONR⁹R¹⁰, CONR⁹Y³, CON (OR⁹) R¹⁰, COR⁹, CN, NR⁹CO₂R¹⁰, NR⁹CONR¹⁰R¹¹, phthalimido, heteroaryl, substituted heteroaryl, heterocycloalkyl, NR⁹R¹⁰ , NR⁹SO₂R¹⁰ , NR⁹COR¹⁰ , NR⁹COY³, NR⁹COCO₂R¹⁰, NR⁹COCONR¹⁰R¹¹, NR⁹COCOR¹⁰, OR⁹, OCOR⁹, OCOY³, OCO₂R⁹, OCONR⁹R¹⁰, OCOCO₂R⁹, OCOCOR⁹, OCOCONR⁹R¹⁰, OSO₂R⁹, PO (OR⁹)₂, SR⁹, SOR⁹, SO₂R⁹, SO₃R⁹, SO₂NR⁹R¹⁰, Cl, Br, I and the like;
wherein R⁹, R¹⁰ and R¹¹ are independently hydrogen, alkyl, cycloalkyl, alkenyl, alkynyl, cycloalkylalkyl, arylalkyl, substituted arylalkyl, aryl, substituted aryl, heteroaryl, substituted heteroaryl, heteroarylalkyl, substituted heteroarylalkyl or heterocycloalkyl;
Y³ is mono-substituted alkyl or di-substituted alkyl.

The alkyl of the term "mono-substituted alkyl" as used in Y³ includes straight or branched C₁-C₄ alkyl. Typical examples are methyl, ethyl, propyl, 2-propyl, butyl and the like. The substituent of the term "mono-substituted alkyl" as used in Y³ includes CO₂R⁹, CONR⁹R¹⁰, COR⁹, CN, NR⁹CO₂R¹⁰, NR⁹CONR¹⁰R¹¹, phthalimido, NR⁹R¹⁰ , NR⁹SO₂R¹⁰, NR⁹COR¹⁰, OR⁹, OCOR⁹, OCO₂R⁹, OCONR⁹R¹⁰ and the like; wherein R⁹, R¹⁰ and R¹¹ are the same as defined above.

The alkyl of the term "di-substituted alkyl" as used in Y³ includes straight or branched C₁-C₄ alkyl. Typical examples are methyl, ethyl, propyl, 2-propyl, butyl and the like. The substituent of the term "di-substituted alkyl" as used in Y³ includes CO₂R⁹, CONR⁹R¹⁰ , COR⁹, CN, NR⁹CO₂R¹⁰, NR⁹CONR¹⁰R¹¹, phthalimido, aryl, substitued aryl, heteroaryl, substitued heteroaryl, heterocycloalkyl, NR⁹R¹⁰, NR⁹SO₂R¹⁰, NR⁹COR¹⁰, OR⁹, OCOR⁹, OCO₂R⁹, OCONR⁹R¹⁰ and the like; wherein R⁹, R¹⁰ and R¹¹ are the same as defined above.

The number of substituents of substituted aryl, substituted arylalkyl, substituted heteroaryl or substituted heteroarylalkyl may be permitted to be up to 3, and the substituents include alkyl, halogen, cyano, trifluoromethyl, nitro, hydroxy, mercapto, amino, alkylamino, alkoxy, alkanoyl, alkoxycarbonyl, carboxy, sulfamoyl, carbamoyl, alkylcarbamoyl, alkylthio, alkylsulfinyl, alkylsulfonyl, alkylsulfamoyl, alkylsulfonylamino, acylamino, substitued alkyl, substituted alkenyl, substituted alkynyl and the like;
wherein the alkyl of the term "substituted alkyl" includes straight or branched C₁-C₄ alkyl; and the substituent of the term "substituted alkyl" includes amino, alkylamino, alkoxycarbony, carboxy, carbamoyl and the like. Typical examples of "substituted alkyl" are methoxycarbonylmethyl, carboxymethyl, α-ethoxycarbonylethyl, β -ethoxycarbonylethyl, α-carboxyethyl, β-carboxyethyl, aminomethyl, methylaminomethyl, dimethylaminomethyl, α-aminoethyl, β-aminoethyl, α-methylaminoethyl, β-ethylaminoethyl, α-ethylaminoethyl, carbamoylmethyl and the like. The alkenyl of the term "substituted alkenyl" includes straight or branched C₂-C₅ alkenyl; and the substituent of the term "substituted alkenyl" includes amino, alkylamino, alkoxycarbony, carboxy, carbamoyl and the like. Typical examples of "substituted alkenyl" are methoxycarbonylvinyl, carboxyvinyl, α-ethxycarbonyvinyl, α -carboxyvinyl, 3-aminopropenyl, 3-methylaminopropenyl, 3-diethylaminopropenyl, 4-carbamoylbutenyl and the like. The alkynyl of the term "substituted alkynyl" includes straight or branched C₂-C₅ alkynyl; and the substituent of the term "substituted alkynyl" includes amino, alkylamino, alkoxycarbony, carboxy, carbamoyl and the like. Typical examples of "substituted alkynyl" are methoxycarbonylethynyl, carboxyethynyl, 3-amino-1-propynyl, 3-methylamino-1-propynyl, 3-diethylamino-1-propynyl, 4-carbamoyl-1-butynyl and the like.

The pharmaceutically acceptable salts of the tricyclic nitrogen-containing compound represented by figure 1, 2 or 3, include, for example, salts with inorganic acids such as the hydrochloride, hydrobromide, sulfate, phosfate and the like; salts with organic acids such as the acetate, oxalate, citrate, lactate, tartrate, malonate, fumarate, maleate, mathanesulfonate and the like; salts with inorganic `metals such as the lithium salt, sodium salt, potassium salt, magnesium salt, aluminum salt, barium salt and the like; salts with organic bases such as the ammonium salt, triethylammonium salt, tetrabutylammonium salt, pyridinium salt, pyrrolidinium salt, piperidinium salt and the like. The present invention includes solvates such as the hydrate and the like, of the tricyclic nitrogen-containing compound or the pharmaceutically acceptable salt thereof.

The tricyclic nitrogen-containing compound which is an N-methyl-D-aspartate receptor antagonist has activity in protecting retinal neuronal cells, and is useful as a medicament for treating retinal neuropathy. The tricyclic nitrogen-containing compound preferably treats retinal neuropathy associated with ischemia. Retinal neuropathy as used herein includes for example glaucoma, central retinal artery occlusion, branch retinal artery occlusion, central retinal vein occlusion, branch retinal vein occlusion, ischemic optic neuropathy, diabetic retinopathy, macular degeneration, retinopathy of prematurity and the like.

The medicament of the present invention may be administered orally or parenterally. Pharmaceutical forms for oral administration include for example tablets, pills, capsules, powders, granules, suspensions, emulsions, syrups and the like. Pharmaceutical forms for parenteral administration include for example intravenous injections such as drops, intramuscular injections, subcutaneous injections, percutaneous preparations, intranasal preparations, eye drops and the like.

The solid compositions such as tablets can be prepared by mixing the active compound with pharmaceutically acceptable conventional carriers or excipients such as lactose, sucrose, corn starch or the like; binders such as hydroxypropylcellulose, polyvinylpyrrolidone, hydroxypropylmethylcellulose or the like; disintegrating agents such as sodium carboxymethylcellulose, sodium starch glycolate or the like; lubricants such as stearic acid, magnesium stearate or the like; or preservatives or the like.

For parenteral administration, the active compound can be dissolved or suspended in a physiologically acceptable carrier such as water, saline, oil, dextrose solution or the like, which may contain auxiliary agents such as emulsifiers, stabilizers, salts for influencing osmotic pressure or buffers, if desired.

The dose for administration varies widely depending on the grade of the symptoms, the patient's age, body weight, sex, administration route, and the like. But the tricyclic nitrogen-containing compound is usually administered to an adult in a dose of approximately 1 - 500 mg, preferably 5 - 100 mg in one portion or several portions per day, by the oral route. By the parenteral route, the tricyclic nitrogen-containing compound is usually administered to an adult in a dose of approximately 0.1 - 100 mg, preferably 0.3 - 50 mg in one portion or several portions per day.

### Examples

The present invention is explained below precisely with examples but is, of course, not limited by them.

The neural retina mainly consists of the outer nuclear layer, the inner nuclear layer and a layer of ganglion cells. The inner nuclear cells and the ganglion cells receive blood supply from the central retinal artery. Putting pressure on the anterior chamber of the eye leads to oppression of the central retinal artery at the lamina cribrosa, which is not covered by sclera, and causes ischemic injury in the inner nuclear cells and the layer of ganglion cells.

Injury of the inner nuclear cells is easily evaluated by measuring "thickness of the inner nuclear layer (INL)" which reflects the number of the inner nuclear cells. "Thickness from the outer limiting membrane to the inner limiting membrane (OLM-ILM)"which reflects the whole neural retina, is another good index to study the degree of retinal damage, because the outer and the inner plexiform layers which consist of synaptic connections between axons and dendrites also become thinner with elevated intraocular pressure. We studied a medicament for treating retinal neuropathy which has a protective effect, by measuring these two parameters as index.

### Example 1

### Pharmacological effect in a rat model with elevated intraocular pressure

### [Experimental method]

Male Sprague-Dawley rats (8 weeks old, Charles River Japan Inc.) were purchased and maintained in a cyclic light/dark environment (8:00-20:00 / light term) for one week before the experiments on elevated intraocular pressure. Rats were anesthetized with pentobarbital (40 mg/kg, s.c.) and placed in a stereotaxic frame. A 27-gauge needle connected to a manometer/pump assembly was inserted into the anterior chamber of one eye. The eye was pressured under 160 mm Hg for 90 minutes. The rats were returned to their home cages, and killed by decapitation under deep anesthesia 7 days later. The eyes were immediately enucleated, embedded in Technovite 7100 (registered trademark: Heraeus Kulzer Co.) and sliced into arrow-shape sections of 2 µm thickness including the optic nerve head. The sections were stained with 0.5 % cresyl violet for 5 min, and observed with an optical microscope. The degree of the inner nuclear cell's damage was quantified by measuring the thickness of the inner nuclear layer. Microscopic photographs of 320 magnification were taken at 880 µm away from the optic nerve head in the superior region. The mean thickness from the outer limiting membrane to the inner limiting membrane and that of the inner nuclear layer were determined by averaging three measurements every 75 µm on the photographs.

Test compound was injected twice, intravenously 30 min before increasing intraocular pressure and intraperitoneally immediately after reperfusion.

### [Pharmacological test]

The pharmacological effect of the tricyclic nitrogen-containing compound represented below, which is described in JP 7-2855, A (EP-627434), was examined.

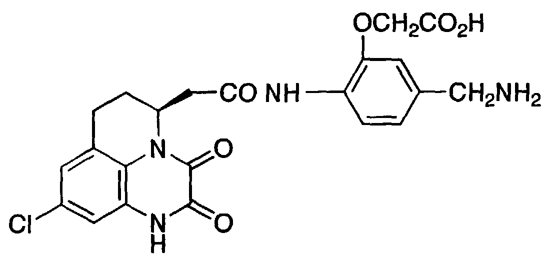

Rats in the test compound group were treated with a 5 mg/kg dosage of the test compound. Rats in the vehicle group were similarly pressured and treated with the same volume of saline (2ml/kg). Rats in the sham operation group were subjected only to insertion of a needle into the anterior chamber of the eye without raising the pressure, and treated with the same volume of saline. Rats in the control group were not subjected to insertion of a needle, and were treated with the same volume of saline.

The results are shown in Tables 1 and 2.

The thickness from the outer limiting membrane to the inner limiting membrane and that of the inner nuclear layer were reduced in the vehicle `group. This result indicates that the elevated intraocular pressure causes the damage in neural retina. On the other hand, in the test compound group, the reduction in these two parameters was significantly smaller than that in the vehicle group. These results demonstrate that the test compound suppressed damage caused in the neural retina by elevated intraocular pressure.

**Table 1**

| Thickness from the outer limiting membrane to the inner limiting membrane (OLM-ILM) | | | | |
|---|---|---|---|---|
| Group | Distance from optic nerve head (µm) | n | Thickness of OLM-ILM ^{a} | |
| | | | (µm) | % of control |
| Control | 880 | 5 | 156.9 ± 6.8 | 100.0 ± 4.3 |
| Sham operation | 880 | 5 | 131.8 ± 3.3 | 84.0 ± 2.1 |
| Vehicle | 880 | 10 | 84.7 ± 2.1^{##} | 156.9 ± 1.3^{##} |
| Test compound | 880 | 9 | 107.9 ± 8.5* | 156.9 ± 5.4* |

| | | | | |
|---|---|---|---|---|
| ∗ P<0.05, Welch test: vs. the vehicle group. | | | | |
| ## P<0.01, Student t-test: vs. the sham operation group | | | | |
| a Mean ± S.E. | | | | |

**Table 2**

| Thickness of the inner nuclear layer (INL) | | | | |
|---|---|---|---|---|
| Group | Distance from optic nerve head (µm) | n | Thickness of INL ^{a} | |
| | | | (µm) | % of control |
| Control | 880 | 5 | 25.5 ± 1.5 | 100.0 ± 5.9 |
| Sham operation | 880 | 5 | 21.6 ± 2.3 | 84.8 ± 8.9 |
| Vehicle | 880 | 10 | 13.8 ± 0.9^{##} | 54.3 ± 3.4^{##} |
| Test compound | 880 | 9 | 22.3 ± 2.2** | 87.4 ± 8.7** |

| | | | | |
|---|---|---|---|---|
| ∗∗ P<0.01, Welch test: vs. the vehicle group | | | | |
| ## P<0.01, Student t-test: vs. the sham operation group | | | | |
| a Mean ± S.E. | | | | |

### Industrial Applicability

The present invention relates to a medicament for treating retinal neuropathy, containing a tricyclic nitrogen-containing compound which is an N-methyl-D-aspartate receptor antagonist or a pharmaceutically acceptable salt thereof.

The present invention may treat glaucoma, central retinal artery occlusion, branch retinal artery occlusion, central retinal vein occlusion, branch retinal vein occlusion, ischemic optic neuropathy, diabetic retinopathy, macular degeneration, retinopathy of prematurity and the like.

## Claims

1. A medicament for treating retinal neuropathy, containing a tricyclic nitrogen-containing compound which is an N-methyl-D-aspartate receptor antagonist or a pharmaceutically acceptable salt thereof.

2. The medicament of Claim 1, wherein the tricyclic nitrogen-containing compound which is an N-methyl-D-aspartate receptor antagonist, is a compound represented by figure 1, 2 or 3: 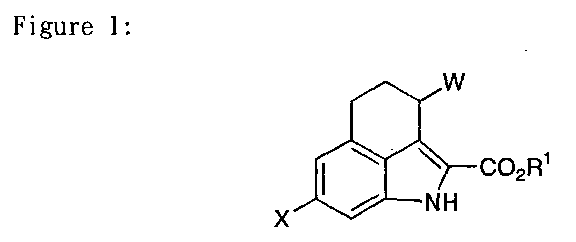 wherein
X is alkyl, halogen or cyano;
R¹ is hydrogen or a carboxyl protecting group;
W is hydrogen, -CO₂R³ⁱ, -CONR³ⁱR⁴ⁱ, -A-CO₂R³ⁱ or -A-CONR³ⁱR⁴ⁱ, wherein -A-is alkylene, and R³ⁱ and R⁴ⁱ are independently hydrogen, alkyl, aryl or substituted aryl. 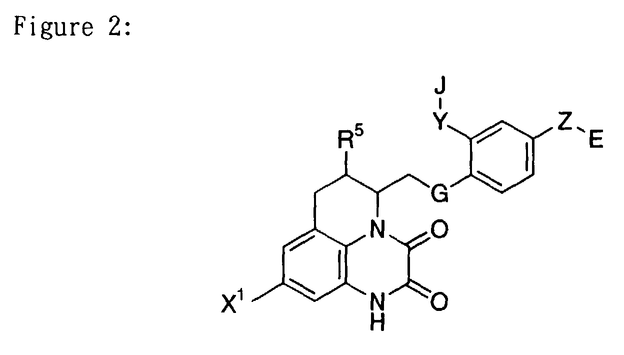
wherein
X¹ is hydrogen, alkyl, halogen, cyano, trifluoromethyl or nitro;
R⁵ is hydrogen, alkyl, cycloalkyl or cycloalkylalkyl;
G is -CONR⁶- or -NR⁶CO-, wherein R⁶ is hydrogen or alkyl;
J is an acidic group or a group which is convertible thereto *in viv*o;
Y is a single bond, alkylene, alkenylene, substituted alkylene or -Y¹-Q-Y²-, wherein Y¹ is a single bond or alkylene, Y² is alkylene, Q is oxygen or sulfur;
E is a basic group or a group which is convertible thereto *in vivo*;
Z is alkylene. 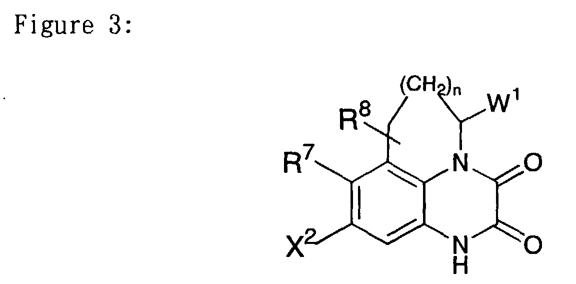
wherein
X² is alkyl, halogen, cyano, trifluoromethyl, nitro, hydroxy, amino, alkylamino, alkoxy, alkanoyl, alkoxycarbonyl, sulfamoyl, carbamoyl, alkylcarbamoyl, alkylthio, alkylsulfinyl, alkylsulfonyl, alkylsulfamoyl, alkylsulfonylamino or acylamino;
R⁷ is hydrogen, alkyl, halogen, cyano, trifluoromethyl, nitro, hydroxy, amino, alkylamino, alkoxy, alkanoyl, alkoxycarbonyl, sulfamoyl, carbamoyl, alkylcarbamoyl, alkylthio, alkylsulfinyl, alkylsulfonyl, alkylsulfamoyl, alkylsulfonylamino or acylamino;
R⁸ is hydrogen, alkyl, cycloalkyl, alkenyl, alkynyl, cycloalkylalkyl, arylalkyl, substituted arylalkyl, aryl or substituted aryl;
W¹ is hydrogen, -CO₂R⁹, -CO₂Y³, -CONR⁹R¹⁰, -CONR⁹Y³, -CON(OR⁹)R¹⁰, - COR⁹, cyano, tetrazolyl or substituted alkyl;
R⁹ and R¹⁰ are independently hydrogen, alkyl, cycloalkyl, alkenyl, alkynyl, cycloalkylalkyl, arylalkyl, substituted arylalkyl, aryl, substituted aryl, heteroaryl, substituted heteroaryl, heteroarylalkyl, substituted heteroarylalkyl or heterocycloalkyl;
Y³ is mono-substituted alkyl or di-substituted alkyl;
n is 0 or 1.

3. The medicament of Claim 2, wherein the tricyclic nitrogen-containing compound which is an N-methyl-D-aspartate receptor antagonist, is a compound represented by figure 1.

4. The medicament of Claim 1, 2 or 3, for treating retinal neuropathy associated with ischemia.

5. The medicament of Claim 1, 2 or 3, for treating glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.

6. A method of treating retinal neuropathy which comprises administering a tricyclic nitrogen-containing compound which is an N-methyl-D-aspartate receptor antagonist or a pharmaceutically acceptable salt thereof.

7. A pharmaceutical composition for treating retinal neuropathy, containing a tricyclic nitrogen-containing compound which is an N-methyl-D-aspartate receptor antagonist or a pharmaceutically acceptable salt thereof, and a pharmaceutically acceptable carrier.

8. Use of a tricyclic nitrogen-containing compound which is an N-methyl-D-aspartate receptor antagonist or a pharmaceutically acceptable salt thereof, in the preparation of a medicament for treating retinal neuropathy.
